(19) Europäisches Patentamt / European Patent Office / Office européen des brevets

(11) **EP 4 172 625 B1**

(12) **EUROPEAN PATENT SPECIFICATION**

(45) Date of publication and mention
of the grant of the patent:
**07.08.2024 Bulletin 2024/32**

(21) Application number: **21721203.4**

(22) Date of filing: **19.04.2021**

(51) International Patent Classification (IPC):
***G01N 33/557*** *(2006.01)*

(52) Cooperative Patent Classification (CPC):
**G01N 33/557**

(86) International application number:
**PCT/IB2021/053195**

(87) International publication number:
**WO 2021/260449 (30.12.2021 Gazette 2021/52)**

(54) **A METHOD FOR DETERMINING KINETIC PARAMETERS OF A REACTION**

VERFAHREN ZUR BESTIMMUNG KINETISCHER PARAMETER EINER REAKTION

PROCEDE DE DETERMINATION DES PARAMETRES CINETIQUES D'UNE REACTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2020 CH 7852020**

(43) Date of publication of application:
**03.05.2023 Bulletin 2023/18**

(73) Proprietor: **Creoptix AG**
**8820 Wädenswil (CH)**

(72) Inventor: **COTTIER, Kaspar**
**8820 Wädenswil (CH)**

(74) Representative: **P&TS SA (AG, Ltd.)**
**Avenue J.-J. Rousseau 4**
**P.O. Box 2848**
**2001 Neuchâtel (CH)**

(56) References cited:
- **MURPHY ANDREW J ET AL: "Surface plasmon resonance characterization of calspermin-calmodulin binding kine", ANALYTICAL BIOCHEMISTRY, vol. 376, no. 1, 30 January 2008 (2008-01-30), pages 61 - 72, XP029236306, ISSN: 0003-2697, DOI: 10.1016/J.AB.2008.01.023**
- **YANG DANLIN ET AL: "Comparison of biosensor platforms in the evaluation of high affinity antibody-antigen binding kinetics", ANALYTICAL BIOCHEMISTRY, ACADEMIC PRESS, AMSTERDAM, NL, vol. 508, 27 June 2016 (2016-06-27), pages 78 - 96, XP029642360, ISSN: 0003-2697, DOI: 10.1016/J.AB.2016.06.024**
- **LIU YUN ET AL: "A Compact Biosensor for Binding Kinetics Analysis of Protein-Protein Interaction", IEEE SENSORS JOURNAL, IEEE SERVICE CENTER, NEW YORK, NY, US, vol. 19, no. 24, 15 December 2019 (2019-12-15), pages 11955 - 11960, XP011753687, ISSN: 1530-437X, [retrieved on 20191204], DOI: 10.1109/JSEN.2019.2938655**

## Description

### Technical domain

The present invention concerns a method of determining the kinetic parameters of a reaction between an analyte and ligands which are attached to a test surface of a flow cell; and in particular to a method which involves the using substantially less of the parts of the binding curve which represent association of the analyte to the ligands, and rather using mainly the parts of a binding curve which represent dissociation of the analyte from the ligands, in order to determine the kinetic parameters.

### Background to the invention

In existing methods for determining the kinetic parameters of a reaction between an analyte and ligands which are attached to a test surface of a flow cell, volumes of sample fluids containing said analyte are consecutively flowed over the test surface. As the volumes of sample fluids containing said analyte are consecutively flowed over the test surface, a sensor is used to measure the amount of analyte which is bound to the ligands on the test surface; a curve, representing the amount of analyte which is bound to the ligands on the test surface, known as a binding curve, is output by the sensor. The binding curve will contain association phases and disassociation phases. An association phase occurs when a volume of sample fluid is passing over the test surface and analyte in the sample fluid is binding to the ligands; the binding curve will show an increase in the amount of analyte which is bound to the ligands on the test surface during the association phase. A dissociation phase occurs after a volume of sample fluid is passing over the test surface, and before the next volume of sample fluid is flowed over the test surface; the binding curve will show a decrease in the amount of analyte which is bound to the ligands on the test surface during the dissociation phase and the previously bound analytes become dissociated (or detached) from the ligands.

The entire binding curve (i.e. all of the association phases and disassociation phases) is then used to determine the kinetic parameters of the reaction between the analyte and ligands on the test surface; typically this is done by identifying a predefined model, which has known kinetic parameters, which best fits to the binding curve; the kinetic parameters of the model which best fits to the binding curve are then considered to be the kinetic parameters of the reaction.

However, the problem with using the entire binding curve to determine the kinetic parameters of the reaction between the analyte and ligands on the test surface, is that it may lead to inaccurate determination of the kinetic parameters of the reaction: existing sensors which are used to measure the amount of analyte which is bound to the ligands on the test surface and output a binding curve, are sensitive to changes in refractive index of the volumes of sample fluid; when a volume of sample fluid containing analyte is flowed over the test surface, changes in the refractive index of the volume of sample fluid can introduce artefacts in the binding curve; these artefacts are present in the association phases of the binding curve; because the entire binding curve (i.e. all of the association phases and disassociation phases) is then used to determine the kinetic parameters of the reaction between the analyte and ligands on the test surface, these artefacts in turn leads to inaccurate determination of the kinetic parameters of the reaction.

It is an aim of the present invention to obviate, or mitigate, at least some of the disadvantages associated with the existing methods in the field. In particular it is an aim of the present invention to provide an improve method for determining the kinetic parameters of a reaction, which is less vulnerable to artefacts in the binding curve created by refractive index change which occur when a volume of sample fluid is flowed over the test surface of a flow cell.

### Summary of the invention

According to the present invention, this aim is achieved by a method having, at least, the steps recited in claim 1. The dependent claims recite favourable, optional, steps which can be performed in various embodiments of the invention.

Advantageously, in the method of the present invention mainly the dissociation phase of the binding curve (i.e. the part of the binding curve corresponding to the period after a volume of sample fluid has been flowed over the test surface, and before the next volume of sample fluid is flowed over the test surface), and not the association phase of binding curve (i.e. the part of the binding curve corresponding to when a volume of sample fluid is flowing over the test surface and analyte in the sample fluid is binding to the ligands), is used to determine kinetic parameters of the reaction.

In particular in a first embodiment only the dissociation phases of the binding curve and no association phase of binding curve are used to determine the kinetic parameters; and in a second embodiment the dissociation phases of the binding curve and only a very small part of the association phases of binding curve are used to determine the kinetic parameters. In the second embodiment the small part of the association phases of the binding curve can be so small that artefacts in those parts of the binding curve have a negligible effect on the determination of the kinetic parameters.

In either embodiment the amount of the association phases of the binding curve which is used to determine the kinetic parameters is reduced compared to the prior art.

Accordingly, in the present invention,artefacts in the binding curve created by refractive index changes which occur when a volume of sample fluid is flowed over the test surface of a flow cell, will have less of an effect on the determination of the kinetic parameters.

**Brief description of the drawings**

Exemplary embodiments of the invention are disclosed in the description and illustrated by the drawings in which:

Figure 1 is a complete binding curve which comprises six association phases and six dissociation phases, which resulted from having flowed, consecutively, respective six volumes of sample fluid containing analyte, over the test surface of the flow cell;

Figure 2 shows the parts of the binding curve of Figure 1 which are in a plurality of predefined interval time periods;

Figure 3 shows a normalized concentration curve which is used to determine said plurality of predefined interval time periods.

**Detailed description of embodiments of the present invention**

According to the present invention there is provided a method for determining the kinetic parameters of a reaction between an analyte and ligands which are attached to a test surface of a flow cell. The method comprises the steps of:

(a) Flowing a first volume of sample fluid (V1), which contains said analyte, over the test surface, between a first point in time ($t_1$) to a second point in time ($t_2$).

(b) flowing a first volume of buffer fluid (Vb1) (which is without analyte), over the test surface, between a third point in time ($t_3$) to a fourth point in time ($t_4$) (the third point in time ($t_3$) may be equal to the second point in time ($t_2$)); most preferably dissociation of at least some analytes from the first volume of sample fluid (V1) which were bound to the ligands on the test surface occurs between the third point in time ($t_3$) to the fourth point in time ($t_4$). In one embodiment the buffer fluid is configured to promote the dissociation of bound analyte from the ligands.

(c) Flowing at least a second volume of sample fluid (V2), which contains said analyte, over the test surface, between a fifth point in time ($t_5$) to a sixth point in time ($t_6$) (the fifth point in time ($t_5$) may be equal to the fourth point in time ($t_4$));

(d) flowing a second volume of buffer fluid (Vb2) (which is without analyte), over the test surface, between a seventh point in time ($t_7$) to a eight point in time ($t_8$) (the seventh point in time ($t_7$) may be equal to the sixth point in time ($t_6$)); most preferably dissociation of at least some analytes from the second volume of sample fluid (V2), which were bound to the ligands on the test surface, occurs between the seventh point in time ($t_7$) and eight point in time ($t_8$). In one embodiment the buffer fluid is configured to promote the dissociation of bound analyte from the ligands.

(e) Using a sensor to measure the binding of analyte to ligands on the test surface to obtain a binding curve.

(f) Using only the parts of the binding curve which are in predefined interval time periods, without using the other parts of the binding curve, to determine the kinetic parameters, as recited in claim 1.

In order to determine the kinetic parameters only the parts of the binding curve which are predefined interval time periods, the following steps are carried out: establishing a normalized concentration curve (c(t)) which describes the concentration of the analyte at the test surface over time; estimating values for the kinetic parameters Rmax, $k_a$, $k_d$, wherein Rmax is a predefined theoretical maximum binding curve value corresponding to a saturation of the ligands, such as for instance when all binding sites of the ligands are occupied by analyte bound to the ligands, $k_a$ is the association rate constant and $k_d$ is the dissociation rate constant; using said normalized concentration curve (c(t)), and said estimated values for the kinetic parameters Rmax, $k_a$, $k_d$, to solve the differential reaction equation:

$$\frac{dR}{dt} = k_a c\left(t\right)\left(R_{max} - R(t)\right) - k_d R(t)$$

in order to obtain a simulated binding curve sbc = R(t).

Once the differential reaction equation has been obtained, then extracting the parts of the simulated binding curve which are in said predefined interval time periods ($tj\Delta$) to provide respective partial simulated binding curves (sbcj).

A partial chi square ($\chi^2$) of the simulated binding curve is then established according to the following equation:

$$\chi^2 = \sum_j \ \sum_i \ \frac{(\mathrm{sbcj}_i - \mathrm{dmbj}_i)^2}{N_{dmbj}}$$

wherein dmbj is the part of the binding curve which is in the j-th predefined interval time period (tj$\Delta$), and wherein sbcj is the j-th partial simulated binding curve and $N_{dmbj}$ is the number of data points in the part of the binding curve which is in the j-th predefined interval time period (tj$\Delta$).

After the partial chi square ($\chi^2$) of the simulated binding curve has been determined then, minimizing said determined partial chi square ($\chi^2$), wherein the values of said kinetic parameters Rmax, $k_a$, $k_d$ which minimize said determined partial chi square ($\chi^2$) define the kinetic parameters of said reaction between the analyte and ligands which are attached to a test surface of the flow cell.

In the preferred embodiment the step of estimating values for the kinetic parameters Rmax, $k_a$, $k_d$, comprises using a Levenberg-Marquard algorithm to find the kinetic parameters which minimize the partial chi square ($\chi^2$).

In a further preferred embodiment the step of estimating values for the kinetic parameters Rmax, $k_a$, $k_d$, comprises using Estimators to find the kinetic parameters which minimize the partial chi square ($\chi^2$).

In the preferred embodiment the method comprises the steps of using a sensor to measure the binding of analyte to ligands on the test surface, continuously from the first point in time ($t_1$) to the ninth point in time ($t_9$) and then extracting the parts of the binding curve which are in said predefined interval time periods; and using only said extracted parts of the binding curve to determine the kinetic parameters.

In another embodiment the method comprises the steps of using a sensor to measure the binding of analyte to ligands on the test surface, continuously between from the first point in time ($t_1$) to the ninth point in time ($t_9$); zeroing the parts of the binding curve which are outside of said predefined interval time periods; and then using only said parts of the binding curve which are not zeroed to determine the kinetic parameters.

### *Exemplary Embodiment 1:*

In the first embodiment the predefined interval time periods are a first and second interval time periods (t1$\Delta$, t2$\Delta$); wherein the first interval time period (t1$\Delta$) occurs between the second point in time ($t_2$) and the fifth point in time ($t_5$); and wherein the second interval time period (t2$\Delta$) occurs between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time (ts)). Advantageously, in this embodiment only the parts of the binding curve which correspond to the dissociation phase are used to determine the kinetic parameters.

In one embodiment the first interval time period (t1$\Delta$) is a portion of the duration of time between the second point in time ($t_2$) and the fifth point in time ($t_5$); and the second interval time period (t2$\Delta$) is a portion of the duration of time between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time (ts)). For example the first interval time period (t1$\Delta$) may be between a predefined time period after the second point in time ($t_2$), until the fifth point in time ($t_5$), or until a predefined time period before the fifth point in time ($t_5$); and the second interval time period (t2$\Delta$) may be between a predefined time period after the sixth point in time ($t_6$) until the eight point in time ($t_8$), or until a predefined time period before the eight point in time ($t_8$). In another embodiment the first interval time period (t1$\Delta$) is defined by the whole time interval between the second point in time ($t_2$) and the fifth point in time ($t_5$); and the second interval time period (t2$\Delta$) is defined by the whole time interval between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time ($t_8$)).

This first embodiment comprises the steps of:

(a) Flowing a first volume of sample fluid (V1), which contains said analyte, over the test surface, between a first point in time ($t_1$) to a second point in time ($t_2$).

(b) flowing a first volume of buffer fluid (Vb1) (which is without analyte), over the test surface, between a third point in time ($t_3$) to a fourth point in time ($t_4$) (the third point in time ($t_3$) may be equal to the second point in time ($t_2$)); most preferably dissociation of at least some analytes from the first volume of sample fluid (V1) which were bound to the ligands on the test surface occurs between the third point in time ($t_3$) to the fourth point in time ($t_4$). In one embodiment the buffer fluid is configured to promote the dissociation of bound analyte from the ligands.

(c) Flowing at least a second volume of sample fluid (V2), which contains said analyte, over the test surface, between a fifth point in time ($t_5$) to a sixth point in time ($t_6$) (the fifth point in time ($t_5$) may be equal to the fourth point in time ($t_4$));

(d) flowing a second volume of buffer fluid (Vb2) (which is without analyte), over the test surface, between a seventh point in time ($t_7$) to a eight point in time ($t_8$) (the seventh point in time ($t_7$) may be equal to the sixth point in time ($t_6$)); most preferably dissociation of at least some analytes from the second volume of sample fluid (V2), which were bound to the ligands on the test surface, occurs between the seventh point in time ($t_7$) and eight point in time ($t_8$). In one embodiment the buffer fluid is configured to promote the dissociation of bound analyte from the ligands.

(e) Using a sensor to measure the binding of analyte to ligands on the test surface, at least during a first and a second interval time period ($t1\Delta$, $t2\Delta$), wherein the first interval time period ($t1\Delta$) occurs between the second point in time ($t_2$) and fifth point in time ($t_5$), and the second interval time period ($t2\Delta$) occurs between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time (ts)) to obtain a binding curve. In one embodiment the first interval time period ($t1\Delta$) is a portion of the duration of time between the second point in time ($t_2$) and the fifth point in time ($t_5$); and the second interval time period ($t2\Delta$) is a portion of the duration of time between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time ($t_8$)). For example the first interval time period ($t1\Delta$) may be between a predefined time period after the second point in time ($t_2$), until the fifth point in time ($t_5$), or until a predefined time period before the fifth point in time ($t_5$); and the second interval time period ($t2\Delta$) may be between a predefined time period after the sixth point in time ($t_6$) until the eight point in time ($t_8$), or until a predefined time period before the eight point in time ($t_8$). In another embodiment the first interval time period ($t1\Delta$) is defined by the whole time interval between the second point in time ($t_2$) and the fifth point in time ($t_5$); and the second interval time period ($t2\Delta$) is defined by the whole time interval between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time ($t_8$)).

(f) Using the part of the binding curve which is in said first and second interval time periods ($t1\Delta$, $t2\Delta$), without using the parts of the binding curve which are between a first point in time ($t_1$) and second point in time ($t_{2a}$) and between the fifth point in time ($t_5$) to the sixth point in time ($t_6$), to determine the kinetic parameters.

In this first embodiment substantially none of the binding curve which are between a first point in time ($t_1$) and second point in time ($t_2$) and between the fifth point in time ($t_5$) and sixth point in time ($t_6$) are used to determine the kinetic parameters. Accordingly, artefacts in the binding curve created by refractive index changes which occur when a volume of sample fluid is flowed over the test surface of a flow cell, will have less of an effect on the determination of the kinetic parameters.

In one embodiment the method comprises the steps of using a sensor to measure the binding of analyte to ligands on the test surface, continuously from the first point in time ($t_1$) to the ninth point in time ($t_9$) and then extracting the parts of the binding curve which are in said first and second interval time periods ($t1\Delta$, $t2\Delta$); and using only said extracted parts of the binding curve to determine the kinetic parameters.

In another embodiment the method comprises the steps of using a sensor to measure the binding of analyte to ligands on the test surface, continuously between from the first point in time ($t_1$) to the ninth point in time ($t_9$); zeroing the parts of the binding curve which are outside of said first and second interval time periods ($t1\Delta$, $t2\Delta$); and then using only said parts of the binding curve which are not zeroed to determine the kinetic parameters.

The step of using the part of the binding curve which is in said first and second interval time periods ($t1\Delta$, $t2\Delta$), without using the parts of the binding curve which are between a first point in time ($t_1$) and second point in time ($t_2$) and between the fifth point in time ($t_5$) and sixth point in time ($t_6$), to determine the kinetic parameters, can be carried out using any known techniques for determining kinetic parameters using a binding curve; those very same techniques can be used in the present invention to determine kinetic parameters, the difference being the techniques are applied to the parts of the binding curve in said first and second interval time periods ($t1\Delta$, $t2\Delta$), and not to the parts of the binding curve which are between a first point in time ($t_1$) and second point in time ($t_2$) and between the fifth point in time ($t_5$) and sixth point in time ($t_6$).

In the most favourable embodiment the step of using the parts of the binding curve which is in said first and second interval time periods ($t1\Delta$, $t2\Delta$), without using the parts of the binding curve which are between a first point in time ($t_1$) and second point in time ($t_2$) and between the fifth point in time ($t_5$) and sixth point in time ($t_6$), to determine the kinetic parameters, preferably comprises the steps of: establishing a normalized concentration curve (c(t)) which describes the concentration of the analyte at the test surface over time (preferably a concentration curve is first established then it is normalized to provide said normalized concentration curve (c(t))); estimating values for the kinetic parameters Rmax, $k_a$, $k_d$, wherein Rmax is a predefined theoretical maximum binding curve value corresponding to a saturation of the ligands corresponding to a saturation of the ligands, such as for instance when all binding sites of the ligands are occupied by analyte bound to the ligands, $k_a$ is the association rate constant and $k_d$ is the dissociation rate constant; using said normalized concentration curve (c(t)), and said estimated values for the kinetic parameters Rmax, $k_a$, $k_d$, to solve the differential reaction equation:

$$\frac{dR}{dt} = k_a c\,(t)\,\left(R_{max} - R(t)\right) - k_d R(t)$$

in order to obtain a simulated binding curve sbc = R(t).

Once the differential reaction equation has been obtained, then extracting the part of the simulated binding curve which is in said first interval time period (t1Δ) to provide a first partial simulated binding curve (sbc1); and extracting the part of the simulated binding curve which is in said second interval time period (t2Δ) to provide a second partial simulated binding curve (sbc2); and determining a partial chi square ($\chi^2$) of the simulated binding curve according to the following equation:

$$\chi^2 = \sum \frac{(\text{sbc1}_i - \text{dmb1}_i)^2}{N_{dmb1}} + \sum \frac{(\text{sbc2}_i - \text{dmb2}_i)^2}{N_{dmb2}}$$

wherein dmb1 is the part of the binding curve which is in said first interval time period (t1Δ) and dmb2 is the part of the binding curve which is in said second interval time period (t1Δ), and wherein sbc1 is the first partial simulated binding curve and sbc2 is the second partial simulated binding curve, and wherein $N_{dmb1}$ is the number of data points of the binding curve which is in said first interval time period (t1Δ) and $N_{dmb2}$ is the number of data points of the binding curve which is in said second interval time period (t2Δ).

After the partial chi square ($\chi^2$) of the simulated binding curve has been determined then, minimizing said determined partial chi square ($\chi^2$), wherein the values of said kinetic parameters Rmax, $k_a$, $k_d$ which minimize said determined partial chi square ($\chi^2$) define the kinetic parameters of said reaction between the analyte and ligands which are attached to a test surface of the flow cell.

In the preferred embodiment the step of estimating values for the kinetic parameters Rmax, $k_a$, $k_d$, comprises using a Levenberg-Marquard algorithm to find the kinetic parameters which minimize the partial chi square ($\chi^2$).

In a further preferred embodiment the step of estimating values for the kinetic parameters Rmax, $k_a$, $k_d$, comprises using Estimators to find the kinetic parameters which minimize the partial chi square ($\chi^2$).

### *Exemplary Embodiment 2:*

In the first embodiment described above, the predefined interval time periods comprise first and second interval time periods (t1Δ, t2Δ); wherein the first interval time period (t1Δ) occurs between the second point in time ($t_2$) and fifth point in time ($t_5$); and wherein the second interval time period (t2Δ) occurs between the sixth point in time ($t_6$) and a ninth point in time ($t_9$) wherein the ninth point in time ($t_9$) occurs sometime after the eight point in time ($t_8$) (or, in another embodiment, the ninth point in time ($t_9$) is equal to the eight point in time ($t_8$)). In this second embodiment, however, the predefined interval time periods are determined from a concentration curve (or from a normalized concentration curve). The second embodiment comprises the steps of:

(a) Flowing a first volume of sample fluid (V1), which contains said analyte, over the test surface, between a first point in time ($t_1$) to a second point in time ($t_2$).

(b) flowing a first volume of buffer fluid (Vb1) (which is without analyte), over the test surface, between a third point in time ($t_3$) to a fourth point in time ($t_4$) (the third point in time ($t_3$) may be equal to the second point in time ($t_2$)); most preferably dissociation of at least some analytes from the first volume of sample fluid (V1) which were bound to the ligands on the test surface occurs between the third point in time ($t_3$) to the fourth point in time ($t_4$). In one embodiment the buffer fluid is configured to promote the dissociation of bound analyte from the ligands.

(c) Flowing at least a second volume of sample fluid (V2), which contains said analyte, over the test surface, between a fifth point in time ($t_5$) to a sixth point in time ($t_6$) (the fifth point in time ($t_5$) may be equal to the fourth point in time ($t_4$));

(d) flowing a second volume of buffer fluid (Vb2) (which is without analyte), over the test surface, between a seventh point in time ($t_7$) to a eight point in time ($t_8$) (the seventh point in time ($t_7$) may be equal to the sixth point in time ($t_6$)); most preferably dissociation of at least some analytes from the second volume of sample fluid (V2), which were bound to the ligands on the test surface, occurs between the seventh point in time ($t_7$) and eight point in time ($t_8$). In one embodiment the buffer fluid is configured to promote the dissociation of bound analyte from the ligands.

(e) Using a sensor to measure the binding of analyte to ligands on the test surface, to obtain a binding curve.

(f) Using the parts of the binding curve which is in a predefined first interval time periods ($t1\Delta$) and second interval time period ($t2\Delta$), to determine the kinetic parameters.

In this second embodiment substantially less of the parts of the binding curve which are between a first point in time ($t_1$) and second point in time ($t_2$) and between the fifth point in time ($t_5$) and sixth point in time ($t_6$) are used to determine the kinetic parameters. Accordingly, artefacts in the binding curve created by refractive index changes which occur when a volume of sample fluid is flowed over the test surface of a flow cell, will have less of an effect on the determination of the kinetic parameters.

In one embodiment the method comprises the steps of using a sensor to measure the binding of analyte to ligands on the test surface, continuously from the first point in time ($t_1$) to the ninth point in time ($t_9$) and then extracting the parts of the binding curve which are in said first and second interval time periods ($t1\Delta$, $t2\Delta$); and using only said extracted parts of the binding curve to determine the kinetic parameters.

In another embodiment the method comprises the steps of using a sensor to measure the binding of analyte to ligands on the test surface, continuously between from the first point in time ($t_1$) to the ninth point in time ($t_9$); zeroing the parts of the binding curve which are outside of said first and second interval time periods ($t1\Delta$, $t2\Delta$); and then using only said parts of the binding curve which are in said first and second interval time periods ($t1\Delta$, $t2\Delta$) (i.e. the parts of the binding curve which are not zeroed) to determine the kinetic parameters.

The second embodiment further comprises a calibration step which may be carried out before steps (a)-(f), or, may be carried out after steps (a)-(e) (before step (f) is carried out), to determine the first and second interval time periods ($t1\Delta$, $t2\Delta$).

In one embodiment the calibration step comprises, establishing a concentration curve which describes the concentration of the analyte at the test surface over time, and then normalizing that concentration curve to provide a normalized concentration curve (c(t)); and then using that normalized concentration curve (c(t)) to determine the first and second interval time periods ($t1\Delta$, $t2\Delta$). In another embodiment the calibration step comprises, establishing a concentration curve which describes the concentration of the analyte at the test surface over time; then using that concentration curve to determine the first and second interval time periods ($t1\Delta$, $t2\Delta$). Also it should be noted that the same steps are carried out to determine the first and second interval time periods ($t1\Delta$, $t2\Delta$) from the normalized concentration curve (c(t)) as are carried out to determine the first and second interval time periods ($t1\Delta$, $t2\Delta$) from the concentration curve (c(t)). In both variations the normalized concentration curve (c(t)) is used to determine the kinetic parameters.

In the present exemplary second embodiment it will be described establishing a concentration curve which describes the concentration of the analyte at the test surface over time, and then normalizing that concentration curve to provide a normalized concentration curve (c(t)); and then using that normalized concentration curve (c(t)) to determine the first and second interval time periods ($t1\Delta$, $t2\Delta$):

In the present exemplary second embodiment in which first and second volumes of sample fluid (V1,V2) are flowed over the test surface of the flow cell in order to establish a binding curve, a first volume of refractive index standard fluid (V1'), which contains a known concentration of a reference molecule, is flowed over the test surface between a first reference point in time ($t'_1$) to a second reference point in time ($t'_2$).

Importantly the rate at which the first volume of refractive index standard fluid (V1') is flowed over the test surface (i.e. the flow rate) is equal to the rate at which the first volume of sample fluid (V1) is flowed over the test surface; also the duration of time between the a first reference point in time ($t'_1$) to a second reference point in time ($t'_2$) is equal to the duration of time between the first point in time ($t_1$) to a second point in time ($t_2$).

After the first volume of refractive index standard fluid (V1'), which contains a known concentration of a reference molecule, has been flowed over the test surface, a first volume of buffer fluid (Vb1') (which is without reference molecules) is flowed over the test surface, between a third reference point in time ($t'_3$) to a fourth reference point in time ($t'_4$) (the third reference point in time ($t'_3$) may be equal to the second reference point in time ($t'z$)).

A second volume of refractive index standard fluid (V2'), which contains a known concentration of a reference molecule, is flowed then over the test surface between a fifth reference point in time ($t'_5$) to a sixth reference point in time ($t'_6$).

Importantly the rate at which the second volume of refractive index standard fluid (V2') is flowed over the test surface (i.e. the flow rate) is equal to the rate at which the second volume of sample fluid (V2) is flowed over the test surface; also the duration of time between the fifth reference point in time ($t'_5$) to a sixth reference point in time ($t'_6$) is equal to the duration of time between the fifth point in time ($t_5$) to a sixth point in time ($t_6$); also the ratio of the concentration of the second volume of refractive index standard fluid (V2') to the concentration of the first volume of refractive index standard fluid (V1'), is equal to the ratio of the concentration of the second volume of sample fluid (V2) to first volume of sample fluid (V1); also the duration of time between the second reference point in time ($t'_2$) and the fifth reference point in time ($t'_5$) is equal to the duration of time between the second point in time ($t_2$) and the fifth reference point in time ($t'_5$).

After the second volume of refractive index standard fluid (V2'), which contains a known concentration of a reference molecule, has been flowed over the test surface flowing, a second volume of buffer fluid (Vb2') (which is without reference molecules) is flowed over the test surface, between a seventh reference point in time ($t_7$) to a eight reference point in time ($t_8$) (the seventh point in time ($t_7$) may be equal to the sixth reference point in time ($t'6$).

The concentration of the reference molecule at the test surface is measured continuously over the period from the first reference point in time ($t'_1$) to a ninth reference point in time ($t'_9$) wherein the ninth reference point in time ($t'_9$) occurs sometime after the eight reference point in time ($t'_8$) (or, in another embodiment, the ninth reference point in time ($t'_9$) is equal to the eight reference point in time ($t'_8$)), so as to obtain a concentration curve (cmc).

The concentration curve (cmc) is then normalized by dividing the concentration curve (cmc) by the maximum value of the concentration curve (max(cmc)), and multiplying the result by a known maximum concentration ($c_{max}$) of analyte in the in the first and second volumes of sample fluid (V1,V2)), as illustrated in the following equation:

$$c(t) = ((cmc) / max(cmc)) * c_{max}$$

to obtain a normalized concentration curve (c(t)).

In this example the concentration (c(V1)) of analyte in the first volume of sample fluid (V1) is equal to the concentration (c(V2)) of analyte in the second volume of sample fluid (V2) (i.e. both first and second volumes of sample fluid (V1,V2) have the same concentration of analyte); if however one of the volumes of sample fluid (V1,V2) would contain a higher concentration of analyte than the other, then the higher concentration value would define $c_{max}$ (i.e. ($c_{max}$ = max (c(V1), c(V2))). It should be understood that the concentration of analyte in a volume of sample fluid can be determined using any suitable technique which is well known in the art.

In order to determine the first and second interval time periods (t1$\Delta$, t2$\Delta$) using the normalized concentration curve (c(t)), a threshold concentration is selected. Most preferably the threshold concentration is determined as a predefined percentage of the maximum value of the normalized concentration curve (c(t)); for example if the maximum value of the normalized concentration curve (c(t)) curve is '200', then the threshold concentration may be selected to be 5% of the maximum value, which in this example would be 5%*200 = 10; so the threshold concentration in this example would be '10'; in another example the threshold concentration may be 2% of the maximum value, which in this example would be 2%*200 = 4, so the threshold concentration in this example would be '4'.

The earliest time interval on the normalized concentration curve (c(t)), from the time when the normalized concentration curve (c(t)) drops below the threshold concentration, until the time when the normalized concentration curve (c(t)) is equal to the threshold concentration, defines the first interval time period (t1$\Delta$). The next time interval on the normalized concentration curve (c(t)) (which occurs sometime after the first interval time period (t1$\Delta$)), from the time when the normalized concentration curve (c(t)) drops below the threshold concentration, until the ninth reference point in time ($t'_9$), defines the second interval time period (t2$\Delta$).

It should be understood that in a variation of this embodiment the concentration curve (not normalized) may alternatively be used to determine the first and second interval time periods (t1$\Delta$, t2$\Delta$); again in this variation of the embodiment a threshold concentration is selected. Most preferably the threshold concentration is determined as a predefined percentage of the maximum value of the concentration curve; for example if the maximum value of the concentration curve curve is '2000', then the threshold concentration may be selected to be 5% of the maximum value, which in this example would be 5%*2000 = 100; so the threshold concentration in this example would be '100'; in another example the threshold concentration may be 2% of the maximum value, which in this example would be 2%*2000 = 40, so the threshold concentration in this example would be '40. The earliest time interval on the concentration curve, from the time when the concentration curve drops below the threshold concentration, until the time when the concentration curve is equal to the threshold concentration, defines the first interval time period (t1$\Delta$). The next time interval on the concentration curve (which occurs sometime after the first interval time period (t1$\Delta$)), from the time when the concentration curve drops below the threshold concentration, until the ninth reference point in time ($t'_9$), defines the second interval time period (t2$\Delta$).

In the above examples, most preferably the refractive index standard fluid preferably comprises a buffer fluid which contains a known concentration of a reference molecule; and the reference molecule (which is present in a known concentration in the refractive index standard fluid) may comprise, for example, DMSO or Glucose. Most preferably the concentration of a reference molecule in the refractive index standard fluid (and thus the concentration of a reference molecule in the first volume of refractive index standard fluid (V1'), and also the concentration of a reference molecules in the second volume of refractive index standard fluid (V2')) is preferably 0.1% v/v, or 0.5% v/v, or 1% v/v, so the refractive index of the refractive index standard fluid is different from the refractive index of first and second volumes of buffer fluid (Vb1',Vb2') which are without a reference molecules.

The step (f) of using the part of the binding curve which is in said first and second interval time periods (t1Δ, t2Δ), to determine the kinetic parameters, can be carried out using any known techniques for determining kinetic parameters using a binding curve; the very same techniques can be used in the present invention to determine kinetic parameters, the difference being the techniques are applied to the parts of the binding curve in said first and second interval time periods (t1Δ, t2Δ).

In the most favourable embodiment the step of using the parts of the binding curve which is in said first and second interval time periods (t1Δ, t2Δ), to determine the kinetic parameters, preferably comprises the steps of, establishing a normalized concentration curve (c(t)) which describes the concentration of the analyte at the test surface over time (if a normalized concentration curve (c(t)) has not already been established; for example the normalized concentration curve (c(t) established in the calibration step may be used) (using any of steps described in the present application for obtaining a normalized concentration curve (c(t))); estimating values for the kinetic parameters Rmax, $k_a$, $k_d$, wherein Rmax is a predefined theoretical maximum binding curve value corresponding to a saturation of the ligands , such as for instance when all binding sites of the ligands are occupied by analyte bound to the ligands, $k_a$ is the association rate constant and $k_d$ is the dissociation rate constant; using said normalized concentration curve (c(t)) determined in said calibration step, and said estimated values for the kinetic parameters Rmax, $k_a$, $k_d$, to solve the differential reaction equation:

$$\frac{dR}{dt} = k_a c\left(t\right)\left(R_{max} - R(t)\right) - k_d R(t)$$

in order to obtain a simulated binding curve sbc = R(t).

Once the differential reaction equation has been obtained, then extracting the part of the simulated binding curve which is in said first interval time period (t1Δ) to provide a first partial simulated binding curve (sbc1); and extracting the part of the simulated binding curve which is in said second interval time period (t2Δ) to provide a second partial simulated binding curve (sbc2); and determining a partial chi square ($\chi^2$) of the simulated binding curve according to the following equation:

$$\chi^2 = \sum \frac{(\text{sbc1}_i - \text{dmb1}_i)^2}{N_{dmb1}} + \sum \frac{(\text{sbc2}_i - \text{dmb2}_i)^2}{N_{dmb2}}$$

wherein dmb1 is the part of the binding curve which is in said first interval time period (t1Δ) and dmb2 is the part of the binding curve which is in said second interval time period (t1Δ), and wherein sbc1 is the first partial simulated binding curve and sbc2 is the second partial simulated binding curve, and wherein $N_{dmb1}$ is the number of data points of the binding curve which is in said first interval time period (t1Δ) and $N_{dmb2}$ is the number of data points of the binding curve which is in said second interval time period (t2Δ).

After the partial chi square ($\chi^2$) of the simulated binding curve has been determined then, minimizing said determined partial chi square ($\chi^2$), wherein the values of said kinetic parameters Rmax, $k_a$, $k_d$ which minimize said determined partial chi square ($\chi^2$) define the kinetic parameters of said reaction between the analyte and ligands which are attached to a test surface of the flow cell.

In the preferred embodiment the step of estimating values for the kinetic parameters Rmax, $k_a$, $k_d$, comprises using a Levenberg-Marquard algorithm to find the kinetic parameters which minimize the partial chi square ($\chi^2$).

In a further preferred embodiment the step of estimating values for the kinetic parameters Rmax, $k_a$, $k_d$, comprises using Estimators to find the kinetic parameters which minimize the partial chi square ($\chi^2$).

*Establishing a normalized concentration curve:*

Each of the embodiments of the present invention involve a step of establishing a concentration curve which describes the concentration of the analyte at the test surface over time. It should be noted that the concentration curve may be established using any suitable known methods in the art.

In the present invention the step of establishing a concentration curve may be done using volumes of refractive index standard fluid, which contains known concentration of a reference molecule, as describe in the second embodiment above.

In another embodiment the concentration curve which describes the concentration of the analyte at the test surface over time, can be established using a mathematical model of the convection and diffusion fluid dynamics transport phenomena.

In yet another embodiment the concentration curve which describes the concentration of the analyte at the test surface over time, can be established using a simplified model not considering transport phenomena: In this embodiment the concentration curve formed by setting the concentration curve to a 'zero' value for time periods before the first point in time ($t_1$), during the first interval time period (t1$\Delta$), and during the second interval time period (t2$\Delta$); the concentration curve set to a constant value of $c_1$ between the first point in time ($t_1$) and the second point in time ($t_2$), wherein $c_1$ is the concentration of analyte at the test surface between the first point in time ($t_1$) and the second point in time ($t_2$) as the first volume of sample fluid (V1) is flowed over the test surface (i.e. $c_1$ is the concentration of the analyte in the first volume of sample fluid); the concentration curve set to a constant value of $c_2$ between the fifth point in time ($t_5$) to a sixth point in time ($t_6$),wherein $c_2$ is the average concentration of analyte at the test surface between the fifth point in time ($t_5$) and the sixth point in time ($t_6$), as the second volume of sample fluid (V2) is flowed over the test surface (i.e.$c_2$ is the concentration of the analyte in the first volume of sample fluid).

Regardless of what steps are taken to establish the concentration curve, in each embodiment of the present invention, after the concentration curve has been established the concentration curve is then normalized to provide a normalized concentration curve ($c(t)$); and then that normalized concentration curve ($c(t)$) is used to determine kinetic parameters in the same manner as described in the embodiment above.

It should be understood that in some embodiments (e.g. the second embodiment) the concentration curve, or the normalized concentration curve, may be further used to determine the predefined interval time periods on the binding curve which are used to determine the kinetic parameters.

### *Exemplary Embodiment with multiple volumes of samples fluids:*

Importantly, the above examples (in particular the first and second embodiments) describe flowing first and second volumes of sample fluid (V1,V2) containing analyte which, over the test surface of the flow cell. However, it should be understood that any number of volumes of sample fluid can be flowed over the test surface of the flow cell (with respective volumes of buffer fluid flowed over the test surface between volumes of sample fluid); the same principles of the present invention apply when multiple volumes of sample fluid (e.g. more than two) are flowed over the test surface of the flow cell. With respect to the second embodiment, during the calibration step, the number of volumes of refractive index standard fluid which are flowed over the test surface of the flow cell, corresponds to the number of volumes of sample fluid containing analyte which, have been, or are to be, flowed over the test surface of the flow cell.

Accordingly, the method of the present invention may comprise the steps of, consecutively flowing a plurality of flowing a plurality of volumes of sample fluid, each of which contains said analyte, over the test surface, wherein there is an interval time period between each respective volume of sample fluid is flowed over the test surface; and flowing a respective volume of buffer fluid (which is without analyte) over the test surface during each respective interval time period; using a sensor to measure the binding of analyte to ligands on the test surface, to obtain a binding curve; using only parts of the binding curve which are in a plurality of predefined interval time periods, to determine the kinetic parameters.

The plurality of predefined interval time periods may be determined in the manner described in first and second embodiments.

As was the case for the first and second embodiments, the method may comprise steps of, using a sensor to measure the binding of analyte to ligands on the test surface, continuously, between the time when the first volume of sample fluid is flowed over the test surface, until the time when the last volume of buffer fluid is flowed over the test surface; extracting the parts of the binding curve which are in said plurality of predefined interval time periods ; and using only said extracted parts of the binding curve to determine the kinetic parameters. In a further embodiment the method comprises extracting the parts of the binding curve which are in said plurality of predefined interval time periods and the part of the binding curve which is after the last volume of sample fluid has flowed over the test surface until the level of binding drops to a predefined threshold level; and using only said extracted parts of the binding curve to determine the kinetic parameters.

Alternatively, said method may comprise the steps of, using a sensor to measure the binding of analyte to ligands on the test surface, continuously, between the time when the first volume of sample fluid is flowed over the test surface, until the time when the last volume of buffer fluid is flowed over the test surface; zeroing the parts of the binding curve which not in said plurality predefined interval time periods; and using only said parts of the binding curve which are not zeroed to determine the kinetic parameters. Optionally, the part of the binding curve which is after the last volume of sample fluid has flowed over the test surface until the level of binding drops to a predefined threshold level may not be zeroed.

As was the case for the first and second embodiments, preferably the buffer fluid has a composition which will enable the buffer to promote the dissociation of analytes, which were bound to the ligands on the test surface, from said ligands. Most preferably the respective volumes of sample fluid preferably are each composed of a buffer fluid comprising analyte; and the respective volumes of buffer fluid preferably are each composed of the same buffer fluid as is in the volumes of sample fluid, but without the analyte.

The step of using only the parts of the binding curve which are which are in said plurality of predefined interval time periods (and optionally, also the part of the binding curve which is after the last volume of sample fluid has flowed over the test surface until the level of binding drops to a predefined threshold level), to determine the kinetic parameters, may be done in the same manner as described above in the first or second embodiments the only difference been that in the first and second embodiments only two volumes of sample fluid (first and second volumes of sample fluid (V1, V2)) and only two interval time periods (first interval time period (t1$\Delta$), and second interval time period (t2$\Delta$), are used, whereas in this second embodiment this concept is extended to multiple volumes of sample fluid and a plurality of predefined interval time periods.

The step of using only the parts of the binding curve which are in said plurality of predefined interval time periods (and optionally, also the part of the binding curve which is after the last volume of sample fluid has flowed over the test surface until the level of binding drops to a predefined threshold level) to determine the kinetic parameters, may be carried out using any known technique for determining kinetic parameters using a binding curve; the very same techniques can be used in the present invention to determine kinetic parameters, the difference being the techniques are applied exclusively to the parts of the binding curve which are in said plurality of predefined interval time periods (and optionally, also the part of the binding curve which is after the last volume of sample fluid has flowed over the test surface until the level of binding drops to a predefined threshold level) only.

Most preferably, in order to determine the kinetic parameters only the parts of the binding curve which are in said plurality of predefined interval time periods (and optionally, also the part of the binding curve which is after the last volume of sample fluid has flowed over the test surface until the level of binding drops to a predefined threshold level), the following steps are carried out: establishing a normalized concentration curve (c(t)) which describes the concentration of the analyte at the test surface over time (using any of the techniques described in the present application); estimating values for the kinetic parameters Rmax, $k_a$, $k_d$, wherein Rmax is a predefined theoretical maximum binding curve value corresponding to a saturation of the ligands, such as for instance when all binding sites of the ligands are occupied by analyte bound to the ligands, $k_a$ is the association rate constant and $k_d$ is the dissociation rate constant; using said normalized concentration curve (c(t)) determined in said calibration step, and said estimated values for the kinetic parameters Rmax, $k_a$, $k_d$, to solve the differential reaction equation:

$$\frac{dR}{dt} = k_a c(t) \left( R_{max} - R(t) \right) - k_d R(t)$$

in order to obtain a simulated binding curve.

Once the differential reaction equation has been obtained, then extracting the parts of the simulated binding curve which are in said plurality of predefined interval time periods (tj$\Delta$) to provide respective partial simulated binding curves (sbcj).

A partial chi square ($\chi^2$) of the simulated binding curve is then established according to the following equation:

$$\chi^2 = \sum_j \sum_i \frac{(\text{sbcj}_i - \text{dmbj}_i)^2}{N_{dmbj}}$$

wherein dmbj is the part of the binding curve which is in the j-th interval time period (tj$\Delta$), and wherein sbcj is the j-th partial simulated binding curve and $N_{dmbj}$ is the number of data points in the part of the binding curve which is in the j-th predefined interval time period (tj$\Delta$).

After the partial chi square ($\chi^2$) of the simulated binding curve has been determined then, minimizing said determined partial chi square ($\chi^2$), wherein the values of said kinetic parameters Rmax, $k_a$, $k_d$ which minimize said determined partial chi square ($\chi^2$) define the kinetic parameters of said reaction between the analyte and ligands which are attached to a test surface of the flow cell.

In the preferred embodiment the step of estimating values for the kinetic parameters Rmax, $k_a$, $k_d$, comprises using a Levenberg-Marquard algorithm to find the kinetic parameters which minimize the partial chi square ($\chi^2$).

In a further preferred embodiment the step of estimating values for the kinetic parameters Rmax, $k_a$, $k_d$, comprises using Estimators to find the kinetic parameters which minimize the partial chi square ($\chi^2$).

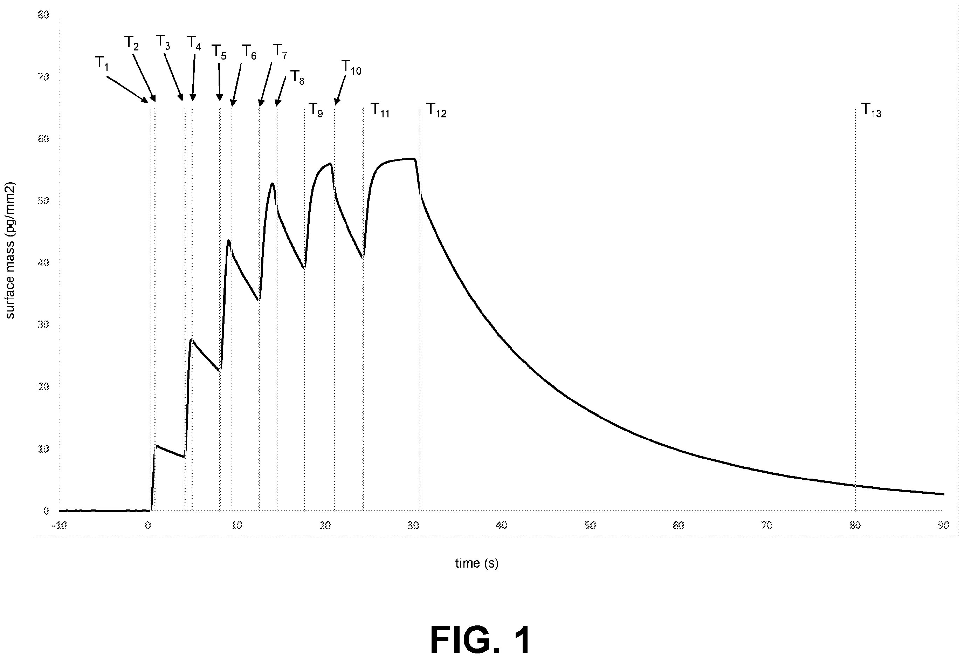

*Description of Figures 1-3:*

Figures 1-3 can be used to demonstrate the principles outlined in the second embodiment above, being applied when multiple number of volumes of sample fluid are flowed over the test surface of the flow cell.

Figure 1 shows an example of complete binding curve which is obtained when six volumes of sample fluid (V1-V6) are consecutively flowed over the test surface of the flow cell. The complete binding curve shows the amount of analyte which is bound to the ligands on the test surface as a first volume of sample fluid (V1) containing analyte is flowed over the test surface (T1-T2); after the first volume of sample fluid containing analyte is flowed over the test surface and before the a second volume of sample fluid is flowed over the test surface (T2-T3), during the time period T2-T3, a first volume of buffer fluid (V'1) (which does not contain analyte) is flowed over the test surface; as a second volume of sample fluid (V2) containing analyte is flowed over the test surface (T3-T4); after the second volume of sample fluid containing analyte is flowed over the test surface and before the a third volume of sample fluid is flowed over the test surface (T4-T5), during the time period T4-T5, a second volume of buffer fluid (V'2) (which does not contain analyte) is flowed over the test surface; as a third volume of sample fluid (V3) containing analyte is flowed over the test surface (T5-T6); after the third volume of sample fluid containing analyte is flowed over the test surface and before the a fourth volume of sample fluid is flowed over the test surface (T6-T7), during the time period T6-T7, a third volume of buffer fluid (V'3) (which does not contain analyte) is flowed over the test surface; as a fourth volume of sample fluid (V4) containing analyte is flowed over the test surface (T7-T8); after the fourth volume of sample fluid containing analyte is flowed over the test surface and before the a fifth volume of sample fluid is flowed over the test surface (T8-T9), during the time period T8-T9, a fourth volume of buffer fluid (V'4) (which does not contain analyte) is flowed over the test surface; as a fifth volume of sample fluid (V5) containing analyte is flowed over the test surface (T9-T10); after the fifth volume of sample fluid containing analyte is flowed over the test surface and before the a sixth volume of sample fluid is flowed over the test surface (T10-T11) during the time period T10-T11, a fifth volume of buffer fluid (V'5) (which does not contain analyte) is flowed over the test surface; as a sixth volume of sample fluid (V6) containing analyte is flowed over the test surface (T11-T12); after the sixth volume of sample fluid containing analyte is flowed over the test surface, until the amount of analyte which is bound to the ligands reduced to a predefined threshold level or until a predefined time period has elapsed (T12-T13), preferably during the time period T12-T13 a sixth volume of buffer fluid (V'6) (which does not contain analyte) is flowed over the test surface .

Preferably the volumes of buffer fluid (V'1-V'6) may preferably have a composition which allows the buffer fluid to promote the dissociation of analytes, which were bound to the ligands on the test surface, from said ligands. Most preferably each of the volumes of sample fluid (V1-V6) are composed of buffer fluid mixed with analyte; each of the volumes of buffer fluid (V'1-V'6) are composed of buffer fluid only (i.e. without the analyte), and most preferable the buffer fluid in each of the volumes of buffer fluid (V'1-V'6) is the same as the buffer fluid in the volumes of sample fluid (V1-V6).

Each of the six volumes of sample fluid (V1-V6) have a known concentration of analyte. The concentration of analyte in each volume can be determined using any known means in the art. Thus the concentration of analyte in each of the six volumes of sample fluid (V1-V6) is known. The volume of sample fluid (V1-V6) with the largest concentration of the analyte is the maximum concentration ($c_{max}$). In this example each of the six volumes of sample fluid (V1-V6) happen to have the same concentration of analyte (so each volume of sample fluid has the a concentration of analyte which equal to the maximum concentration ($c_{max}$)); however in another embodiment the six volumes of sample fluid (V1-V6) may have different concentrations of analyte in which case the maximum concentration ($c_{max}$) is the concentration of analyte in the volume of sample fluid (V1-V6) with the largest concentration.

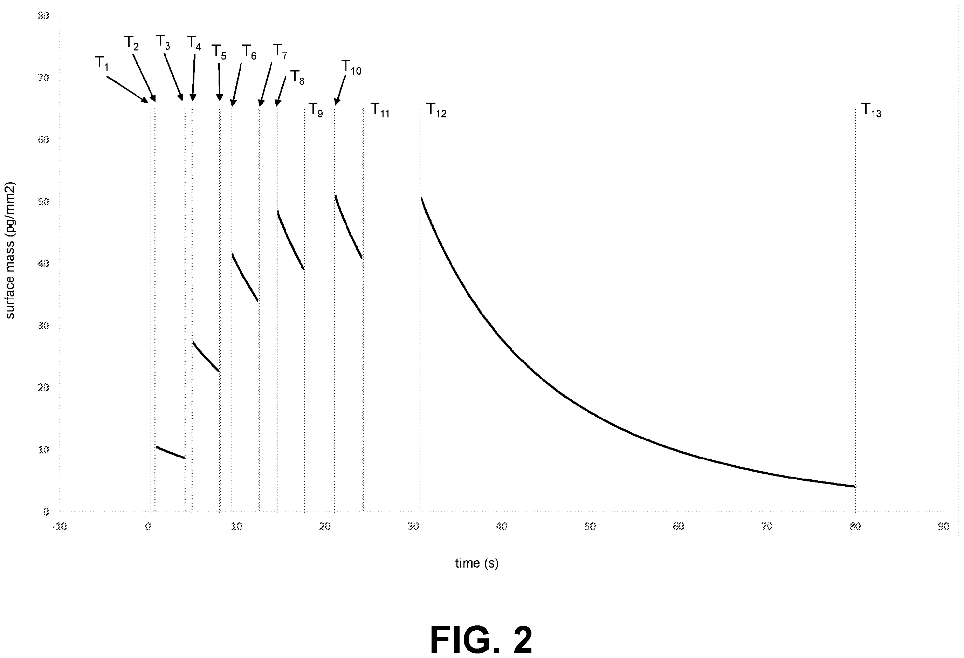

Figure 2 shows a plurality of predefined interval time periods of the binding curve of Figure 1 extracted. It should be understood that Figure 2 could alternatively be considered to show a plurality of predefined interval time periods of the binding curve of Figure 1, with the other parts of the binding curve have been 'zeroed'. Either way, in the present invention Figure 2 illustrates the part of the binding curve which are used, exclusively, in the present invention to determine the kinetic parameters (e.g. in step (e) of the first embodiment).

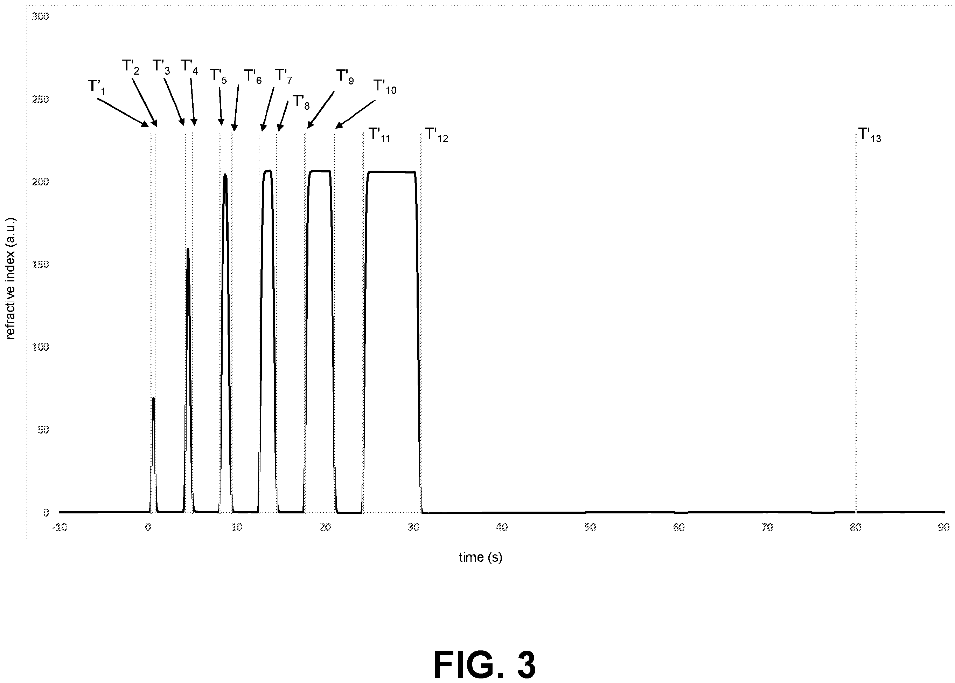

It should be understood that the predefined interval time periods are determined, preferably from a concentration curve or from a normalized concentration curve. Figure 3 shows a normalized concentration curve which was used to determine the predefined interval time periods; these predefined interval time periods need to be determined to know which parts of the binding curve of Figure 1 to extract in order to form the curve shown in Figure 2.

In this case six volumes of sample fluid (V1-V6) are consecutively flowed over the test surface of the flow cell, in order to establish a binding curve (Figure 1), so a corresponding six volumes of refractive index standard fluid, which contains a known concentration of reference molecules, are flowed over the test surface in order to obtain the normalized concentration curve in Figure 3.

The normalized concentration curve in Figure 3 obtained after, a first volume of refractive index standard fluid (V"1) is flowed over the test surface; after the first volume of refractive index standard fluid is flowed over the test surface and before a second volume of refractive index standard fluid is flowed over the test surface, during this time period, a first volume of buffer fluid (V'1) (which does not contain reference molecules) is flowed over the test surface; as a second volume of refractive index standard fluid (V"2) is flowed over the test surface; after the second volume of refractive index standard fluid is flowed over the test surface and before a third volume of refractive index standard fluid is flowed over the test surface, during this time period, a second volume of buffer fluid (V'2) (which does not contain reference molecules) is flowed over the test surface; as a third volume of refractive index standard fluid (V"3) is flowed over the test surface; after the third volume of refractive index standard fluid is flowed over the test surface and before a fourth volume of refractive index standard fluid is flowed over the test surface, during this time period, a third volume of buffer fluid (V'3) (which does not contain reference molecules) is flowed over the test surface; as a fourth volume of refractive index standard fluid (V"4) is flowed over the test surface; after the fourth volume of refractive index standard fluid is flowed over the test surface and before a fifth volume of refractive index standard fluid is flowed over the test surface, during this time period a fourth volume of buffer fluid (V'4) (which does not contain reference molecules) is flowed over the test surface; as a fifth volume of refractive index standard fluid (V"5) is flowed over the test surface; after the fifth volume of refractive index standard fluid is flowed over the test surface and before a sixth volume of refractive index standard fluid is flowed over the test surface, during this time period, a fifth volume of buffer fluid (V'5) (which does not contain reference molecules) is flowed over the test surface; as a sixth volume of refractive index standard fluid (V"6) is flowed over the test surface; after the sixth volume of refractive index standard fluid is flowed over the test surface, until the concentration of the reference molecule at the test surface reduces to a predefined threshold level or until a predefined time period has elapsed, preferably during this time period a sixth volume of buffer fluid (V'6) (which does not contain reference molecules) is flowed over the test surface. Measuring the concentration of reference molecules at the test surface while each of the afore-mentioned steps are carried out (preferably from before the first volume of sample fluid (V1) is flowed over the test surface, until the end of last step when the sixth volume of buffer fluid (V'6) is flowed over the test surface until the concentration of the reference molecule at the test surface reduces to a predefined threshold level or until a predefined time period has elapsed, results in a concentration curve (cmc).

Importantly the rate at which the respective volumes of refractive index standard fluid are flowed over the test surface (i.e. the flow rate) is equal to the rate at which the respective six volumes of sample fluid (V1-V6) are flowed over the test surface. Also the ratio of the concentrations of the volumes of refractive index standard fluid (V1"-V"6) is equal to the ration of the concentrations of the volumes of sample fluids (V1-V6).

The concentration curve (cmc) is then normalized by dividing the concentration curve (cmc)) by the maximum value of the concentration curve (max(cmc)) and multiplying the result by the known maximum concentration ($c_{max}$) of analyte in the respective six volumes of sample fluid (V1-V6) as illustrated in the following equation:

$$c(t) = ((cmc) \, / \, max(cmc)) * c_{max}$$

to provide a normalized concentration curve (c(t)) shown in Figure 3.

In order to determine each of the plurality of predefined interval time periods from the normalized concentration curve (c(t)) shown in Figure 3 a threshold concentration is selected; in this example the threshold concentration is 5% of the maximum value of the normalized concentration curve (c(t)); since the maximum value of the normalized concentration curve (c(t)) is '200', in this example the threshold concentration is '10' (i.e. 5%*200 = 10). The time intervals where the normalized concentration curve (c(t)), drops to below the level of '10', until is raises again to be equal to, or above, '10' defines the plurality of predefined interval time periods. Referring to the normalized concentration curve (c(t)) shown in Figure 3: at time 1'2 the normalized concentration curve (c(t)), drops to below the level of '10', at time $T'_3$ the normalized concentration curve (c(t)), raises to '10, thus $T'_2$-$T'_3$ defines one of the predefined interval time periods; at time $T'_4$ the normalized concentration curve (c(t)), drops to below the level of '10', at time $T'_5$ the normalized concentration curve (c(t)), raises to '10, thus $T'_4$-$T'_5$ defines another one of the predefined interval time periods; at time $T'_6$ the normalized concentration curve (c(t)), drops to below the level of '10', at time $T'_7$ the normalized concentration curve (c(t)), raises to '10, thus $T'_6$-$T'_7$, defines another one of the predefined interval time periods; at time $T'_8$ the normalized concentration curve (c(t)), drops to below the level of '10', at time $T'_9$ the normalized concentration curve (c(t)), raises to '10, thus $T'_8$-$T'_9$ defines another one of the predefined interval time periods; at time $T'_{10}$ the normalized concentration curve (c(t)), drops to below the level of '10', at time $T'_{11}$ the normalized concentration curve (c(t)), raises to '10, thus $T'_{10}$-$T'_{11}$ defines another one of the predefined interval time periods; at time $T'_{12}$ the normalized concentration curve (c(t)), drops to below the level of '10', at time $T'_{13}$ a predefined time period has elapsed, thus $T'_{12}$-$T'_{13}$ defines the last of the predefined interval time periods.

The parts of the binding curve shown in Figure 1, which occur at times corresponding to the predefined interval time periods $T'_2$-$T'_3$, $T'_4$-$T'_5$, $T'_6$-$T'_7$, $T'_8$-$T'_9$, $T'_{10}$-$T'_{11}$, $T'_{12}$-$T'_{13}$ are then extracted to provide the binding curve shown in Figure 2, which is used to determine the kinetic parameters Rmax, $k_a$, $k_d$ in the manner describe above in each of the embodiments of the present invention.

Various modifications and variations to the described embodiments of the invention will be apparent to those skilled in the art without departing from the scope of the invention as defined in the appended claims. Although the invention has been described in connection with specific preferred embodiments, it should be understood that the invention as claimed should not be unduly limited to such specific embodiment.

**Claims**

1. A method for determining the kinetic parameters of a reaction between an analyte and ligands which are attached to a test surface of a flow cell, the method comprising the steps of,

   (a) flowing a first volume of sample fluid (V1), which contains said analyte, over the test surface, between a first point in time ($t_1$) to a second point in time ($t_2$);
   (b) flowing a first volume of buffer fluid (Vb1), which is without analyte, over the test surface, between a third point in time ($t_3$) to a fourth point in time ($t_4$);
   (c) flowing at least a second volume of sample fluid (V2), which contains said analyte, over the test surface, between a fifth point in time ($t_5$) to a sixth point in time ($t_6$);
   (d) flowing at least a second volume of buffer fluid (Vb2), which is without analyte, over the test surface, between a seventh point in time ($t_7$) to a eight point in time ($t_8$);
   (e) using a sensor to measure the binding of analyte to ligands on the test surface to obtain a binding curve;
   (f) using only the parts of the binding curve which are in predefined interval time periods, without using the other parts of the binding curve, to determine the kinetic parameters,
   wherein the predefined interval time periods comprise a first and second interval time periods ($t1\Delta$, $t2\Delta$), and wherein the first interval time period ($t1\Delta$) occurs between the second point in time (tz) and fifth point in time ($t_5$); and wherein the second interval time periods ($t2\Delta$) occurs between the sixth point in time ($t_6$) and a ninth point in time ($t_9$) wherein the ninth point in time ($t_9$) occurs sometime after the eight point in time ($t_8$), or, the ninth point in time ($t_9$) is equal to the eight point in time ($t_8$).

2. A method according to claim 1, wherein the first interval time period ($t1\Delta$) is a portion of the duration of time between the second point in time (tz) and the fifth point in time ($t_5$); and the second interval time period ($t2\Delta$) is a portion of the duration of time between the sixth point in time ($t_6$) and a ninth point in time ($t_9$); or
   wherein the first interval time period ($t1\Delta$) is defined by the whole time interval between the second point in time (tz) and the fifth point in time ($t_5$); and the second interval time period ($t2\Delta$) is defined by the whole time interval between the sixth point in time ($t_6$) and a ninth point in time ($t_9$).

3. A method according to claim 1 further comprising a calibration step to determine the predefined interval time periods; wherein the calibration step comprises, establishing a concentration curve; and using said concentration curve to determine the predefined interval time periods.

4. A method according to claim 3 wherein the step of using said concentration curve to determine the predefined interval time periods, comprises,

   selecting a threshold concentration;
   identifying the time instants when the concentration curve is equal to the threshold concentration;
   wherein each respective predefined interval time period is defined by a period between a time instant when the concentration curve is at the threshold concentration, said concentration curve showing a decrease in concentration before said time instant, and, a next time instant when the concentration curve is at the threshold concentration, said concentration curve showing an increase in concentration before said next time instant.

5. A method according to claim 4 wherein the step of selecting a threshold concentration comprises,

   identifying the maximum value of the concentration curve;
   selecting a percentage value, wherein the threshold concentration is defined by the maximum value of the concentration curve multiplied by said percentage value.

6. A method according to claim 1 further comprising a calibration step to determine the predefined interval time periods; wherein the calibration step comprises, establishing a concentration curve; normalizing said concentration curve to provide a normalized concentration curve (c(t)); and then using that normalized concentration curve (c(t)) to determine the predefined interval time periods.

7. A method according to claim 6 wherein the step of using said normalized concentration curve to determine the predefined interval time periods, comprises,

selecting a threshold concentration;
identifying the time instants when the normalized concentration curve is equal to the to the threshold concentration;
wherein each respective predefined interval time period is defined by a period between a time instant when the normalized concentration curve is at the threshold concentration, said normalized concentration curve showing a decrease in concentration before said time instant, and, a next time instant when the normalized concentration curve is at the threshold concentration, said normalized concentration curve showing an increase in concentration before said next time instant.

8. A method according to claim 7 wherein the step of selecting a threshold concentration comprises,
identifying the maximum value of the normalized concentration curve;
selecting a percentage value, wherein the threshold concentration is defined by the maximum value of the normalized concentration curve multiplied by said percentage value.

9. A method according to any one of the preceding claims comprising,

using a sensor to measure the binding of analyte to ligands on the test surface, continuously from the first point in time ($t_1$), or before the first point in time ($t_1$), until the eight point in time ($t_8$) or after the eight point in time ($t_8$);
extracting the parts of the binding curve which are in said predefined interval time periods;
using only said extracted parts of the binding curve to determine the kinetic parameters.

10. A method according to any one of the preceding claims comprising,
using a sensor to measure the binding of analyte to ligands on the test surface, continuously from the first point in time ($t_1$), or before the first point in time ($t_1$), until the eight point in time ($t_8$) or after the eight point in time ($t_8$); zeroing the parts of the binding curve which are outside of said predefined interval time periods;
using only said parts of the binding curve which are not zeroed to determine the kinetic parameters.

11. A method according to any one of the preceding claims comprising the steps of,

consecutively flowing a plurality of volumes of sample fluid, each of which contains said analyte, over the test surface, wherein there is an interval time period between each respective volume of sample fluid is flowed over the test surface;
flowing a respective volume of buffer fluid, which is without analyte, over the test surface during each respective interval time period;
using a sensor to measure the binding of analyte to ligands on the test surface, to obtain a binding curve;
using only parts of the binding curve which are in a plurality of predefined interval time periods, to determine the kinetic parameters.

12. A method according to any one of the preceding claims, wherein the step of using only the parts of the binding curve which are in predefined interval time periods, without using the other parts of the binding curve, to determine the kinetic parameters, comprises the steps of,

establishing a normalized concentration function c(t), which describes the concentration of the analyte at the test surface over time;
estimating values for kinetic parameters Rmax, $k_a$, $k_d$, wherein Rmax is a predefined maximum response value corresponding to a saturation of the ligands on the test surface, $k_a$ is the association rate constant and $k_d$ is the dissociation rate constant;
using said normalized concentration curve (c(t)) and the estimated values for the kinetic parameters Rmax, $k_a$, $k_d$, solve the differential reaction equation:

$$\frac{dR}{dt} = k_a c\,(t) \left( R_{max} - R(t) \right) - k_d R(t)$$

in order to obtain a simulated binding curve;

extracting parts of the simulated binding curve which are in said plurality of predefined interval time periods (tj$\Delta$) to provide respective partial simulated binding curves (sbcj);

determining a partial chi square ($\chi^2$) of the simulated binding curve according to the following equation:

$$\chi^2 = \sum_j \sum_i \frac{(\text{sbcj}_i - \text{dmbj}_i)^2}{N_{dmbj}}$$

wherein dmbj is the part of the binding curve which is in the j-th predefined interval time period (tj$\Delta$), and wherein sbcj is the j-th partial simulated binding curve and $N_{dmbj}$ is the number of data points in the part of the binding curve which is in the j-th predefined interval time period (tj$\Delta$);

minimizing said determined partial chi square ($\chi^2$), wherein the values of said kinetic parameters Rmax, $k_a$, $k_d$ which minimize said determined partial chi square ($\chi^2$) define the kinetic parameters of said reaction between the analyte and ligands which are attached to a test surface of the flow cell.

13. A method according to claim 12 wherein the step of establishing a concentration function c(t), which describes the concentration of the analyte at the test surface over time, comprises using a Levenberg-Marquard algorithm to find the kinetic parameters which minimize the partial chi square ($\chi^2$).

14. A method according to claim 12 or 13 wherein the step of estimating values for the kinetic parameters Rmax, $k_a$, $k_d$, comprises using Estimators to find the kinetic parameters which minimize the partial chi square ($\chi^2$).

**Patentansprüche**

1. Verfahren zur Bestimmung der kinetischen Parameter einer Reaktion zwischen einem Analyten und Liganden, die an eine Testoberfläche einer Durchflusszelle gebunden sind, wobei das Verfahren die Schritte umfasst:

(a) Fließenlassen eines ersten Volumens an Probenfluid (V1), das den Analyten enthält, über die Testoberfläche zwischen einem ersten Zeitpunkt ($t_1$) und einem zweiten Zeitpunkt ($t_2$);
(b) Fließenlassen eines ersten Volumens an Pufferfluid (Vb1), das ohne Analyt ist, über die Testoberfläche zwischen einem dritten Zeitpunkt ($t_3$) und einem vierten Zeitpunkt ($t_4$);
(c) Fließenlassen von mindestens einem zweiten Volumen an Probenfluid (V2), das den Analyten enthält, über die Testoberfläche zwischen einem fünften Zeitpunkt ($t_5$) und einem sechsten Zeitpunkt ($t_6$);
(d) Fließenlassen von mindestens einem zweiten Volumen an Pufferfluid (Vb2), das ohne Analyt ist, über die Testoberfläche zwischen einem siebten Zeitpunkt ($t_7$) und einem achten Zeitpunkt ($t_8$);
(e) Verwenden eines Sensors zum Messen der Bindung von Analyt an Liganden auf der Testoberfläche, um eine Bindungskurve zu erhalten;
(f) Verwenden nur der Teile der Bindungskurve, die in vordefinierten Intervallzeitperioden liegen, ohne die anderen Teile der Bindungskurve zu verwenden, um die kinetischen Parameter zu bestimmen,
wobei die vordefinierten Intervallzeitperioden eine erste und eine zweite Intervallzeitperiode (t1$\Delta$, t2$\Delta$) umfassen, und wobei die erste Intervallzeitperiode (t1$\Delta$) zwischen dem zweiten Zeitpunkt ($t_2$) und dem fünften Zeitpunkt ($t_5$) liegt; und wobei die zweiten Intervallzeitperioden (t2$\Delta$) zwischen dem sechsten Zeitpunkt ($t_6$) und einem neunten Zeitpunkt ($t_9$) liegen, wobei der neunte Zeitpunkt ($t_9$) zu einer Zeit nach dem achten Zeitpunkt ($t_8$) liegt oder der neunte Zeitpunkt ($t_9$) gleich dem achten Zeitpunkt ($t_8$) ist.

2. Verfahren nach Anspruch 1, wobei die erste Intervallzeitperiode (t1$\Delta$) ein Anteil der Zeitdauer zwischen dem zweiten Zeitpunkt ($t_2$) und dem fünften Zeitpunkt ($t_5$) ist; und die zweite Intervallzeitperiode (t2$\Delta$) ein Anteil der Zeitdauer zwischen dem sechsten Zeitpunkt ($t_6$) und einem neunten Zeitpunkt ($t_9$) ist; oder wobei die erste Intervallzeitperiode (t1$\Delta$) durch das gesamte Zeitintervall zwischen dem zweiten Zeitpunkt ($t_2$) und dem fünften Zeitpunkt ($t_5$) definiert ist; und die zweite Intervallzeitperiode (t2$\Delta$) durch das gesamte Zeitintervall zwischen dem sechsten Zeitpunkt ($t_6$) und einem neunten Zeitpunkt ($t_9$) definiert ist.

3. Verfahren nach Anspruch 1, des Weiteren umfassend einen Kalibrierschritt, um die vordefinierten Intervallzeitperioden zu bestimmen;
wobei der Kalibrierschritt Erstellen einer Konzentrationskurve und Verwenden der Konzentrationskurve zum Bestimmen der vordefinierten Intervallzeitperioden umfasst.

**4.** Verfahren nach Anspruch 3, wobei der Schritt des Verwendens der Konzentrationskurve, um die vordefinierten Intervallzeitperioden zu bestimmen, umfasst:

Auswählen einer Schwellenwertkonzentration;
Identifizieren der Zeitmomente, wenn die Konzentrationskurve gleich der Schwellenwertkonzentration ist;
wobei jede jeweilige vordefinierte Intervallzeitperiode durch eine Periode zwischen einem Zeitmoment, wenn die Konzentrationskurve an der Schwellenwertkonzentration ist, wobei die Konzentrationskurve vor dem Zeitmoment eine Abnahme der Konzentration zeigt, und einem nächsten Zeitmoment, wenn die Konzentrationskurve an der Schwellenwertkonzentration ist, wobei die Konzentrationskurve vor dem nächsten Zeitmoment einen Anstieg der Konzentration zeigt, definiert ist.

**5.** Verfahren nach Anspruch 4, wobei der Schritt des Auswählens einer Schwellenwertkonzentration umfasst:

Identifizieren des Maximalwerts der Konzentrationskurve;
Auswählen eines prozentualen Werts, wobei die Schwellenwertkonzentration durch den Maximalwert der Konzentrationskurve, multipliziert mit dem prozentualen Wert, definiert ist.

**6.** Verfahren nach Anspruch 1, des Weiteren umfassend einen Kalibrierschritt, um die vordefinierten Intervallzeitperioden zu bestimmen;

wobei der Kalibrierschritt Erstellen einer Konzentrationskurve; Normalisieren der Konzentrationskurve, um eine normalisierte Konzentrationskurve (c(t)) bereitzustellen; und dann Verwenden jener normalisierten Konzentrationskurve (c(t)), um die vordefinierten Intervallzeitperioden zu bestimmen, umfasst.

**7.** Verfahren nach Anspruch 6, wobei der Schritt des Verwendens der normalisierten Konzentrationskurve, um die vordefinierten Intervallzeitperioden zu bestimmen, umfasst:

Auswählen einer Schwellenwertkonzentration;
Identifizieren der Zeitmomente, wenn die normalisierte Konzentrationskurve gleich der Schwellenwertkonzentration ist;
wobei jede jeweilige vordefinierte Intervallzeitperiode durch eine Periode zwischen einem Zeitmoment, wenn die normalisierte Konzentrationskurve an der Schwellenwertkonzentration ist, wobei die normalisierte Konzentrationskurve vor dem Zeitmoment eine Abnahme der Konzentration zeigt, und einem nächsten Zeitmoment, wenn die normalisierte Konzentrationskurve an der Schwellenwertkonzentration ist, wobei die normalisierte Konzentrationskurve vor dem nächsten Zeitmoment einen Anstieg der Konzentration zeigt, definiert ist.

**8.** Verfahren nach Anspruch 7, wobei der Schritt des Auswählens einer Schwellenwertkonzentration umfasst: Identifizieren des Maximalwerts der normalisierten Konzentrationskurve; Auswählen eines prozentualen Werts, wobei die Schwellenwertkonzentration durch den Maximalwert der normalisierten Konzentrationskurve, multipliziert mit dem prozentualen Wert, definiert ist.

**9.** Verfahren nach einem der vorhergehenden Ansprüche, umfassend:

Verwenden eines Sensors zum Messen der Bindung des Analyten an Liganden auf der Testoberfläche kontinuierlich von dem ersten Zeitpunkt ($t_1$), oder vor dem ersten Zeitpunkt ($t_1$), bis zum achten Zeitpunkt ($t_8$) oder nach dem achten Zeitpunkt ($t_8$); Extrahieren der Teile der Bindungskurve, die in diesen vordefinierten Intervallzeitperioden liegen;
Verwenden nur der extrahierten Teile der Bindungskurve zum Bestimmen der kinetischen Parameter.

**10.** Verfahren nach einem der vorhergehenden Ansprüche, umfassend:

Verwenden eines Sensors zum Messen der Bindung des Analyten an Liganden auf der Testoberfläche kontinuierlich von dem ersten Zeitpunkt ($t_1$), oder vor dem ersten Zeitpunkt ($t_1$), bis zum achten Zeitpunkt ($t_8$) oder nach dem achten Zeitpunkt ($t_8$); Nullen der Teile der Bindungskurve, die außerhalb dieser vordefinierten Intervallzeitperioden liegen;
Verwenden nur jener Teile der Bindungskurve, die nicht genullt sind, zum Bestimmen der kinetischen Parameter.

**11.** Verfahren nach einem der vorhergehenden Ansprüche, des Weiteren umfassend die Schritte:

nacheinander Fließenlassen einer Vielzahl von Volumina an Probenfluid, wovon jedes den Analyten enthält, über die Testoberfläche, wobei eine Intervallzeitperiode zwischen jedem jeweiligen Volumen von Probenfluid liegt, das über die Testoberfläche fließen gelassen wird;

Fließenlassen eines jeweiligen Volumens an Pufferfluid, das ohne Analyt ist, über die Testoberfläche während jeder jeweiligen Intervallzeitperiode;

Verwenden eines Sensors zum Messen der Bindung von Analyt an Liganden auf der Testoberfläche, um eine Bindungskurve zu erhalten;

Verwenden von nur Teilen der Bindungskurve, die in einer Vielzahl von vordefinierten Intervallzeitperioden liegen, um die kinetischen Parameter zu bestimmen.

12. Verfahren nach einem der vorhergehenden Ansprüche, wobei der Schritt des Verwendens nur der Teile der Bindungskurve, die in vordefinierten Intervallzeitperioden liegen, ohne die anderen Teile der Bindungskurve zu verwenden, um die kinetischen Parameter zu bestimmen, die Schritte umfasst:

Erstellen einer normalisierten Konzentrationsfunktion c(t), die die Konzentration des Analyten an der Testoberfläche über die Zeit beschreibt;

Schätzen von Werten für kinetische Parameter Rmax, $k_a$, $k_d$, wobei Rmax ein vordefinierter Maximalantwortwert ist, der einer Sättigung der Liganden auf der Testoberfläche entspricht, $k_a$ die Assoziationsratenkonstante ist, und $k_d$ die Dissoziationsratenkonstante ist;

Verwenden der normalisierten Konzentrationskurve (c(t)) und der geschätzten Werte für die kinetischen Parameter Rmax, $k_a$, $k_d$, um die Differentialreaktionsgleichung:

$$\frac{dR}{dt} = k_a c\,(t)\left(R_{max} - R(t)\right) - k_d R(t)$$

zu lösen, um die simulierte Bindungskurve zu erhalten; Extrahieren von Teilen der simulierten Bindungskurve, die in der Vielzahl der vordefinierten Intervallzeitperioden (tj$\Delta$) liegen, um jeweilige partielle simulierte Bindungskurven (sbcj) bereitzustellen; Bestimmen eines partiellen Chi-Quadrats ($X^2$) der simulierten Bindungskurve gemäß der folgenden Gleichung:

$$\chi^2 = \sum_j \sum_i \frac{(\mathrm{sbcj}_i - \mathrm{dmbj}_i)^2}{N_{dmbj}}$$

wobei dmbj das Teil der Bindungskurve ist, das in der j. vordefinierten Intervallzeitperiode (tj$\Delta$) liegt, und wobei sbcj die j. partielle simulierte Bindungskurve ist, und $N_{dmbj}$ die Anzahl der Datenpunkte in dem Teil der Bindungskurve ist, das in der j. vordefinierten Intervallzeitperiode (tj$\Delta$) liegt;

Minimieren des bestimmten partiellen Chi-Quadrats ($X^2$), wobei die Werte der kinetischen Parameter Rmax, $k_a$, $k_d$, die das bestimmte partielle Chi-Quadrat ($X^2$) minimieren, die kinetischen Parameter der Reaktion zwischen dem Analyten und Liganden definieren, die an eine Testoberfläche der Durchflusszelle gebunden sind.

13. Verfahren nach Anspruch 12, wobei der Schritt des Erstellens einer Konzentrationsfunktion c(t), die die Konzentration des Analyten an der Testoberfläche im Zeitverlauf beschreibt, Verwenden eines Levenberg-Marquard-Algorithmus umfasst, um die kinematischen Parameter aufzufinden, die das partielle Chi-Quadrat ($X^2$) minimieren.

14. Verfahren nach Anspruch 12 oder 13, wobei der Schritt des Schätzens von Werten für die kinetischen Parameter Rmax, $k_a$, $k_d$, Verwenden von Estimatoren umfasst, um die kinetischen Parameter aufzufinden, die das partielle Chi-Quadrat ($X^2$) minimieren.

**Revendications**

1. Procédé destiné à déterminer les paramètres cinétiques d'une réaction entre un analyte et des ligands qui sont attachés à une surface d'essai d'une cellule d'écoulement, le procédé comprenant les étapes suivantes :

(a) écoulement d'un premier volume de fluide échantillonné (V1), qui contient ledit analyte, sur la surface d'essai, entre un premier point temporel ($t_1$) et un deuxième point temporel ($t_2$) ;

(b) écoulement d'un premier volume de fluide tampon (Vb1), qui est sans analyte, sur la surface d'essai, entre un troisième point temporel ($t_3$) et un quatrième point temporel ($t_4$) ;

(c) écoulement d'au moins un deuxième volume de fluide échantillonné (V2), qui contient ledit analyte, sur la surface d'essai, entre un cinquième point temporel ($t_5$) et un sixième point temporel ($t_6$) ;

(d) écoulement d'au moins un deuxième volume de fluide tampon (Vb2), qui est sans analyte, sur la surface d'essai, entre un septième point temporel ($t_7$) et un huitième point temporel (te) ;

(e) utilisation d'un capteur pour mesurer la liaison de l'analyte aux ligands sur la surface d'essai, afin d'obtenir une courbe de liaison ;

(f) utilisation uniquement des parties de la courbe de liaison qui se trouvent dans des intervalles de temps prédéfinis, sans utilisation des autres parties de la courbe de liaison, pour déterminer les paramètres cinétiques, dans lequel les intervalles de temps prédéfinis comprennent des premier et deuxième intervalles de temps ($t1\Delta$, $t2\Delta$), et dans lequel le premier intervalle de temps ($t1\Delta$) se situe entre le deuxième point temporel ($t_2$) et le cinquième point temporel ($t_5$) ; et dans lequel le deuxième intervalle de temps ($t2\Delta$) se situe entre le sixième point temporel ($t_6$) et un neuvième point temporel ($t_9$), le neuvième point temporel ($t_9$) se situant un peu après le huitième point temporel (te), ou le neuvième point temporel ($t_9$) étant égal au huitième point temporel ($t_8$).

2. Procédé selon la revendication 1, dans lequel le premier intervalle de temps ($t1\Delta$) est une partie de la durée entre le deuxième point temporel ($t_2$) et le cinquième point temporel ($t_5$) ; et le deuxième intervalle de temps ($t2\Delta$) est une partie de la durée entre le sixième point temporel ($t_6$) et un neuvième point temporel ($t_9$) ; ou dans lequel le premier intervalle de temps ($t1\Delta$) est défini par l'intervalle de temps total entre le deuxième point temporel ($t_2$) et le cinquième point temporel ($t_5$) ; et le deuxième intervalle de temps ($t2\Delta$) est défini par l'intervalle de temps total entre le sixième point temporel ($t_6$) et un neuvième point temporel ($t_9$) .

3. Procédé selon la revendication 1 comprenant en outre une étape d'étalonnage pour déterminer les intervalles de temps prédéfinis ;
dans lequel l'étape d'étalonnage comprend l'établissement d'une courbe de concentration, et l'utilisation de ladite courbe de concentration pour déterminer les intervalles de temps prédéfinis.

4. Procédé selon la revendication 3 dans lequel l'étape d'utilisation de ladite courbe de concentration pour déterminer les intervalles de temps prédéfinis comprend :

la sélection d'une concentration seuil ;
l'identification des instants où la courbe de concentration est égale à la concentration seuil ;
dans lequel chaque intervalle de temps prédéfini respectif est défini par une période entre un instant où la courbe de concentration est à la concentration seuil, ladite courbe de concentration présentant une diminution de concentration avant ledit instant, et un instant suivant où la courbe de concentration est à la concentration seuil, ladite courbe de concentration présentant une augmentation de concentration avant ledit instant suivant.

5. Procédé selon la revendication 4 dans lequel l'étape de sélection d'une concentration seuil comprend :

l'identification de la valeur maximale de la courbe de concentration ;
la sélection d'une valeur de pourcentage, la concentration seuil étant définie par la valeur maximale de la courbe de concentration multipliée par ladite valeur de pourcentage.

6. Procédé selon la revendication 1 comprenant en outre une étape d'étalonnage pour déterminer les intervalles de temps prédéfinis ;
dans lequel l'étape d'étalonnage comprend l'établissement d'une courbe de concentration, la normalisation de ladite courbe de concentration pour obtenir une courbe de concentration normalisée ($c(t)$), puis l'utilisation de cette courbe de concentration normalisée ($c(t)$) pour déterminer les intervalles de temps prédéfinis.

7. Procédé selon la revendication 6 dans lequel l'étape d'utilisation de ladite courbe de concentration normalisée pour déterminer les intervalles de temps prédéfinis comprend :

la sélection d'une concentration seuil ;
l'identification des instants où la courbe de concentration normalisée est égale à la concentration seuil ;
dans lequel chaque intervalle de temps prédéfini respectif est défini par une période entre un instant où la courbe de concentration normalisée est à la concentration seuil, ladite courbe de concentration normalisée présentant une diminution de concentration avant ledit instant, et un instant suivant où la courbe de concentration normalisée est à la concentration seuil,

ladite courbe de concentration normalisée présentant une augmentation de concentration avant ledit instant suivant.

8. Procédé selon la revendication 7 dans lequel l'étape de sélection d'une concentration seuil comprend :

l'identification de la valeur maximale de la courbe de concentration normalisée ;
la sélection d'une valeur de pourcentage, la concentration seuil étant définie par la valeur maximale de la courbe de concentration normalisée multipliée par ladite valeur de pourcentage.

9. Procédé selon l'une quelconque des revendications précédentes comprenant :

l'utilisation d'un capteur pour mesurer la liaison de l'analyte aux ligands sur la surface d'essai, de façon continue depuis le premier point temporel ($t_1$), ou avant le premier point temporel ($t_1$), jusqu'au huitième point temporel (te) ou après le huitième point temporel (te) ;
l'extraction des parties de la courbe de liaison qui se trouvent dans lesdits intervalles de temps prédéfinis ;
l'utilisation uniquement desdites parties extraites de la courbe de liaison pour déterminer les paramètres cinétiques.

10. Procédé selon l'une quelconque des revendications précédentes comprenant :

l'utilisation d'un capteur pour mesurer la liaison de l'analyte aux ligands sur la surface d'essai, de façon continue depuis le premier point temporel ($t_1$), ou avant le premier point temporel ($t_1$), jusqu'au huitième point temporel (te) ou après le huitième point temporel (te) ;
la mise à zéro des parties de la courbe de liaison qui se trouvent à l'extérieur desdits intervalles de temps prédéfinis ;
l'utilisation uniquement desdites parties de la courbe de liaison qui ne sont pas mises à zéro pour déterminer les paramètres cinétiques.

11. Procédé selon l'une quelconque des revendications précédentes comprenant les étapes suivantes :

écoulement consécutif d'une pluralité de volumes de fluide échantillonné, dont chacun contient ledit analyte, sur la surface d'essai, un intervalle de temps existant entre chaque volume respectif de fluide échantillonné que l'on fait s'écouler sur la surface d'essai ;
écoulement d'un volume respectif de fluide tampon, qui est sans analyte, sur la surface d'essai pendant chaque intervalle de temps respectif ;
utilisation d'un capteur pour mesurer la liaison de l'analyte aux ligands sur la surface d'essai, afin d'obtenir une courbe de liaison ;
utilisation uniquement des parties de la courbe de liaison qui se trouvent dans une pluralité d'intervalles de temps prédéfinis pour déterminer les paramètres cinétiques.

12. Procédé selon l'une quelconque des revendications précédentes, dans lequel l'étape d'utilisation uniquement des parties de la courbe de liaison qui se trouvent dans des intervalles de temps prédéfinis, sans utilisation des autres parties de la courbe de liaison, pour déterminer les paramètres cinétiques, comprend les étapes suivantes :

établissement d'une fonction de concentration normalisée $c(t)$, qui décrit la concentration de l'analyte au niveau de la surface d'essai dans le temps ;
estimation de valeurs pour des paramètres cinétiques $R_{max}$, $k_a$, $k_d$, $R_{max}$ étant une valeur de réponse maximale prédéfinie correspondant à une saturation des ligands sur la surface d'essai, $k_a$ étant la constante de vitesse d'association et $k_d$ étant la constante de vitesse de dissociation ;
utilisation de ladite courbe de concentration normalisée ($c(t)$) et des valeurs estimées pour les paramètres cinétiques $R_{max}$, $k_a$, $k_d$, pour résoudre l'équation différentielle de réaction :

$$\frac{dR}{dt} = k_a c(t)\big(R_{max} - R(t)\big) - k_d R(t)$$

afin d'obtenir une courbe de liaison simulée ; extraction des parties de la courbe de liaison simulée qui se trouvent dans ladite pluralité d'intervalles de temps prédéfinis (tjΔ) pour obtenir des courbes de liaison simulées partielles respectives (*sbcj*) ; détermination d'un chi carré partiel ($\chi^2$) de la courbe de liaison simulée selon l'équation suivante :

$$\chi^2 = \sum_j \sum_i \frac{(sbcj_i - dmbj_i)^2}{N_{dmbj}}$$

dans laquelle *dmbj* est la partie de la courbe de liaison qui se trouve dans le j$^{\text{ième}}$ intervalle de temps prédéfini (tjΔ), et dans laquelle *sbcj* est la j$^{\text{ième}}$ courbe de liaison simulée partielle et $N_{dmbj}$ est le nombre de points de données dans la partie de la courbe de liaison qui se trouve dans le j$^{\text{ième}}$ intervalle de temps prédéfini (tjΔ) ; minimisation dudit chi carré partiel ($\chi^2$) déterminé, dans lequel les valeurs desdits paramètres cinétiques $R_{max}$, $k_a$, $k_d$ qui minimisent ledit chi carré partiel ($\chi^2$) déterminé définissent les paramètres cinétiques de ladite réaction entre l'analyte et les ligands qui sont attachés à une surface d'essai de la cellule d'écoulement.

13. Procédé selon la revendication 12 dans lequel l'étape d'établissement d'une fonction de concentration *c*(*t*), qui décrit la concentration de l'analyte au niveau de la surface d'essai dans le temps, comprend l'utilisation d'un algorithme de Levenberg-Marquard pour trouver les paramètres cinétiques qui minimisent le chi carré partiel ($\chi^2$).

14. Procédé selon la revendication 12 ou 13 dans lequel l'étape d'estimation de valeurs pour les paramètres cinétiques $R_{max}$, $k_a$, $k_d$ comprend l'utilisation d'estimateurs pour trouver les paramètres cinétiques qui minimisent le chi carré partiel ($\chi^2$).

EP 4 172 625 B1

FIG. 2

FIG. 3

EP 4 172 625 B1